(19) Europäisches Patentamt
European Patent Office
Office européen des brevets

(11) Numéro de publication: **0 550 745 B1**

(12) # FASCICULE DE BREVET EUROPEEN

(45) Date de publication du fascicule du brevet: **20.09.95**

(51) Int. Cl.$^6$: **A61K 7/48**, A61K 7/06

(21) Numéro de dépôt: **92917233.6**

(22) Date de dépôt: **24.07.92**

(86) Numéro de dépôt internationale :
**PCT/FR92/00734**

(87) Numéro de publication internationale :
**WO 93/01797 (04.02.93 93/04)**

(54) **COMPOSITION COSMETIQUE HUILEUSE CONTENANT COMME AGENT EPAISSISSANT UNE ASSOCIATION DE DEUX COPOLYMERES ET CONTENANT FACULTATIVEMENT UN CORRECTEUR DE RHEOLOGIE AMPHIPHILE.**

(30) Priorité: **25.07.91 FR 9109438**

(43) Date de publication de la demande:
**14.07.93 Bulletin 93/28**

(45) Mention de la délivrance du brevet:
**20.09.95 Bulletin 95/38**

(84) Etats contractants désignés:
**AT BE CH DE DK ES FR GB GR IT LI NL SE**

(56) Documents cités:
**EP-A- 0 140 274**
**EP-A- 0 406 042**
**FR-A- 2 305 967**
**EP-A- 0 268 164**
**FR-A- 2 238 474**
**FR-A- 2 305 969**

(73) Titulaire: **L'OREAL**
**14, rue Royale**
**F-75008 Paris (FR)**

(72) Inventeur: **MONDET, Jean**
**34, rue Daniel-Fery**
**F-93700 Drancy (FR)**
Inventeur: **LION, Bertrand**
**2, rue Denis-Papin**
**F-93190 Livry-Gargan (FR)**
Inventeur: **CANDAU, Didier**
**5, allée Nicolas-de-Stael**
**F-77000 Melun (FR)**
Inventeur: **SIMON, Pascal**
**97 bis, rue Auber**
**F-94400 Vitry-sur-Seine (FR)**

(74) Mandataire: **Stalla-Bourdillon, Bernard**
**CABINET NONY & CIE**
**29, rue Cambacérès**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

L'invention a pour objet l'utilisation en association, comme agents épaississants dans une composition cosmétique huileuse, de deux copolymères comprenant des motifs distincts.

On sait que de nombreuses compositions cosmétiques destinées notamment à être appliquées sur la peau, les lèvres, les cils et les cheveux se présentent sous la forme de solutions huileuses ou sous la forme d'émulsions. La préparation de ces compositions nécessite généralement l'épaississement de la phase huileuse de façon notamment à en faciliter l'application.

Il est également nécessaire d'épaissir la phase huileuse lorsqu'on veut obtenir une composition sous la forme d'un gel, par exemple d'un gel anhydre. La formulation sous forme de gel anhydre est utile lorsque les substances présentes dans la composition sont sensibles à l'humidité et/ou à l'oxygène de l'air. On sait en outre que les huiles ont des propriétés cosmétiques très intéressantes (notamment nettoyantes, démaquillantes, émollientes), mais que leur utilisation n'est pas commode notamment, car elles sont trop fluides. En fait, leur application est peu agréable lorsqu'elles ne sont pas présentées sous forme de composition huileuse épaissie ou de gel.

Une méthode classique d'épaississement des compositions huileuses consiste à incorporer une cire dans la phase huileuse. Toutefois, les compositions épaissies à l'aide de cire ont un toucher généralement considéré comme désagréable.

On a également utilisé des techniques d'épaississement des huiles fondées sur l'incorporation de silices, de bentones ou de sels métalliques d'acides gras, (par exemple sels d'aluminium) de dérivés d'estérification de sucres, (par exemple palmitate de dextrine) etc...

Le document EP-A-0 406 042 décrit une composition cosmétique contenant comme agent épaississant un polymère.

Toutefois, aucun de ces procédés ne permet d'obtenir une composition possédant à la fois la transparence et la consistance d'un gel.

On a maintenant découvert qu'il est possible d'obtenir un épaississement qui est notamment capable de concilier ces exigences, dans la réalisation de compositions cosmétiques, quelle que soit la nature de l'huile ou du mélange d'huiles utilisé.

On a également découvert que les propriétés rhéologiques et cosmétiques des compositions ainsi obtenues peuvent être modifiées et améliorées, si on le souhaite, grâce à l'utilisation d'un correcteur de rhéologie, et notamment de certains agents amphiphiles, comme cela sera précisé ci-après.

Les gels obtenus selon l'invention n'ont pas une texture cassante, c'est-à-dire qu'ils peuvent être pris facilement avec le doigt en adhérence sur celui-ci et n'ont pas non plus une texture filante, c'est-à-dire qu'ils ne filent pas en s'écoulant comme le fait par exemple un miel liquide.

Un des avantages de l'utilisation de polymères pour l'épaississement des compositions cosmétiques est qu'ils ne passent pas la barrière cutanée, de sorte qu'il n'y a pas lieu de redouter une toxicité systémique.

Un autre avantage de l'utilisation des polymères est qu'il est possible d'obtenir un épaississement important avec des quantités relativement faibles d'agent épaississant.

En outre, l'utilisation d'un système reposant sur l'association de deux polymères est que chacun des polymères, avant le mélange, donne des solutions fluides, ce qui facilite les techniques de préparation, en évitant notamment des temps de dissolution importants et en évitant la présence d'impuretés insolubles dans le milieu après mélange.

La présente invention a donc pour objet l'utilisation en association comme agents épaississants des huiles dans une composition cosmétique comprenant une phase huileuse, d'au moins un premier copolymère comprenant des motifs dérivés d'au moins un monomère lipophile et des motifs A dérivés d'au moins un monomère hydrophile comprenant au moins un groupement acide carboxylique ou sulfonique, et d'au moins un second copolymère comportant des motifs dérivés d'au moins un monomère lipophile et des motifs B dérivés d'au moins un monomère hydrophile comprenant au moins un groupement amine, amide, alcool ou éther, lesdits premier et second copolymères ayant une masse moléculaire non inférieure à 100 000 environ.

Dans des modes de réalisation particuliers, l'utilisation selon l'invention peut encore présenter les caractéristiques suivantes, prises isolément ou, le cas échéant, en combinaison :

- dans lesdits premier et second copolymères, la proportion pondérale des motifs dérivés du monomère lipophile est au moins égale à 50 % ;
- dans lesdits premier et second copolymères, la proportion pondérale des motifs A et des motifs B, respectivement, est au moins égale à 2 % ;

- lesdits motifs A sont dérivés d'au moins un monomère choisi parmi les acides carboxyliques et sulfoniques insaturés ;
- lesdits acides carboxyliques insaturés comprennent :
  - les monoacides carboxyliques insaturés,
  - et les diacides carboxyliques insaturés, leurs monoesters et leurs monoamides ;
- lesdits monoacides carboxyliques insaturés sont choisis parmi l'acide acrylique, l'acide méthacrylique et l'acide crotonique, et/ou lesdits diacides sont choisis parmi l'acide maléïque et l'acide itaconique, et/ou lesdits monoesters ou monoamides dérivent respectivement d'alcools ou d'amines ayant de 1 à 22 atomes de carbone ;
- lesdits acides sulfoniques insaturés sont choisis parmi l'acide acrylamido-2 méthyl-2 propanesulfonique et le méthacrylate de 2-sulfoéthyle ;
- lesdits motifs B sont dérivés d'au moins un monomère hydrophile comprenant un groupement amine répondant à la formule :

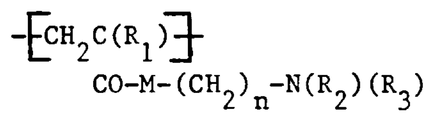

dans laquelle :
- M représente -O- ou -NH-,
- $R_1$ représente -H ou $-CH_3$,
- n est un nombre de 2 à 20,
- $R_2$ et $R_3$ représente indépendamment -H ou un groupement hydrocarboné ayant 1 à 4 atomes de carbone ;
- lesdits motifs B sont dérivés de monomères choisis parmi le méthacrylate de diméthylaminoéthyle, l'acrylate de diéthylaminoéthyle et le N-diméthylaminopropyl méthacrylamide ;
- lesdits motifs B sont dérivés d'au moins un monomère hydrophile insaturé comprenant un groupement amide, choisis parmi l'acrylamide, le méthacrylamide, le N,N-diméthylacrylamide, la N-vinyl pyrrolidone et le diacétone acrylamide ;
- les motifs, dérivés de monomères lipophiles, qui sont présents dans les deux types de copolymères utilisés selon l'invention, peuvent être des motifs dérivés de monomères utilisés de façon habituelle, notamment en cosmétologie, lorsqu'on veut introduire des motifs lipophiles dans un polymère. Ces monomères sont par exemple des esters dérivés d'acides insaturés et d'alcools gras à longue chaîne, notamment des acrylates ou méthacrylates de stéaryle ou de lauryle ;
- lesdits copolymères ont une masse moléculaire au moins égale à 200 000 ;
- les proportions pondérales relatives desdits premier et second copolymères dans ladite association, sont dans l'intervalle de 10:90 à 90:10 et en particulier dans l'intervalle de 25:75 à 75:25 ;
- la concentration totale desdits premier et second copolymères dans ladite composition est inférieure à 10 % et en particulier inférieure à 5 % en poids ;
- la concentration totale desdits copolymères doit être suffisante pour obtenir le degré d'épaississement désiré qui est bien entendu variable selon les cas ; généralement la concentration totale desdits copolymères est supérieure à 0,1 % en poids, et peut varier en particulier entre 0,5 et 5 % ; généralement, lorsque la concentration totale desdits copolymères atteint 2 à 3 % environ, on obtient une huile gélifiée (à texture de gel).

Dans l'utilisation selon l'invention, la phase huileuse contient généralement au moins 55%, et de préférence au moins 75 % en poids d'huile(s).

Pour obtenir, selon l'invention, des compositions cosmétiques épaissies, on opère de préférence par mélange d'une solution de l'un des polymères préalablement dissous dans une partie de l'huile ou du mélange d'huiles à épaissir, avec une solution de l'autre polymère dans l'autre partie de l'huile ou du mélange d'huiles à épaissir.

De préférence, on ajoute les autres ingrédients liposolubles de la composition, l'on dissout chaque copolymère dans une partie de l'huile, ou du mélange d'huiles, à épaissir, et l'on mélange ensuite les deux solutions obtenues.

A l'exception de cette particularité, les compositions cosmétiques épaissies obtenues selon l'invention sont préparées selon les méthodes usuelles.

Lorsque les compositions finales sont des émulsions, en particulier des émulsions eau-dans-l'huile (E/H), on prépare bien entendu la phase huileuse, comme indiqué ci-dessus, avant de préparer l'émulsion avec une phase aqueuse.

On a en outre découvert qu'il est possible d'améliorer les propriétés des compositions huileuses épaissies ainsi obtenues, lorsque cela est estimé nécessaire ou souhaitable, à l'aide d'un agent correcteur de rhéologie. C'est le cas notamment lorsque le gel obtenu est trop visqueux et a tendance à être cassant, ou lorsqu'il est moins visqueux mais peu souple et très filant. On a découvert notamment que certains agents amphiphiles non ioniques sont susceptibles d'améliorer les propriétés rhéologiques et cosmétiques des compositions de l'invention, en leur conférant un comportement rhéologique de type pseudo-plastique qui permet de développer à bas gradient de vitesse des contraintes compatibles avec la prise du produit et avec son étalement. On entend par contrainte, la force qu'oppose le produit à une sollicitation extérieure, et par gradient de vitesse, la variation spatiale de la vitesse de déformation du produit. On obtient ainsi des textures gélifiées préhensiles et non filantes. Sans introduction d'agent amphiphile, les contraintes développées à bas gradient risquent d'être soit élevées, caractérisant un gel solide et cassant, soit faibles, caractérisant un gel filant. On obtient donc, grâce à l'invention, des textures particulièrement bien adaptées à des produits cosmétiques, notamment des gels préhensiles et non filants.

Les compositions de l'invention peuvent donc contenir un agent correcteur de rhéologie. L'agent correcteur de rhéologie est notamment un agent amphiphile non ionique ayant une valeur de HLB comprise entre 12 et 40 environ. Cet agent amphiphile est de préférence utilisé sous forme hydratée, c'est-à-dire en présence d'une certaine quantité d'eau et, éventuellement, en présence d'un alcool soluble dans l'eau. L'alcool soluble dans l'eau est par exemple l'éthanol, l'isopropanol, ou un polyol tel que le glycérol, le propylène glycol, le butanediol-1,3, le sorbitol, le glucose, etc... Les agents amphiphiles non ioniques utilisables comme agents correcteurs de rhéologie selon l'invention peuvent être choisis notamment parmi :

- les esters d' acides gras et de sorbitan polyoxyéthyléné,
- les esters d'acides gras et de glycérol polyoxyéthyléné,
- les esters d'acides gras et de propylène glycol polyoxyéthyléné,
- les alkyl éthers polyoxyéthylénés ou polyoxypropylénés,
- les alkyl phényl éthers polyoxyéthylénés ou polyoxypropylénés,
- les alcools de Guerbet polyoxyéthylénés.

Les composés amphiphiles mentionnés ci-dessus sont des composés connus.

On rappelle que les alcools de Guerbet sont des alcools de formule :

$RCH_2CH_2CH(R)CH_2OH$

dans laquelle R représente notamment un groupement alkyle à longue chaîne, par exemple un groupement octyl-2 dodécyle. Les éthers polyoxyéthylénés de ces alcools sont préparés selon les méthodes usuelles.

Les agents amphiphiles non ioniques sont introduits en quantité suffisante pour conférer les propriétés rhéologiques souhaitées. Cette quantité suffisante peut être déterminée dans chaque cas par de simples expériences de routine. Généralement cette quantité représente de 1 à 10 % en poids, le plus souvent de 4 à 6 % en poids, par rapport au poids de la phase huileuse.

Il convient de noter que la quantité d'eau mentionnée ici est la quantité d'eau utilisée pour l'hydratation de l'agent amphiphile, c'est-à-dire l'eau introduite en même temps que l'agent amphiphile non ionique. La composition finale peut naturellement contenir des quantités plus importantes d'eau lorsqu'elle se présente sous la forme d'une émulsion.

Les compositions de l'invention, contenant l'agent correcteur de rhéologie mentionné ci-dessus, sont donc notamment celles dont la phase huileuse contient (% en poids, total 100 %) :

- mélange épaississant de copolymères : 0,5-10 % (de préférence 2-4 %)
- agent amphiphile non ionique : 1-10 % (de préférence 4-6 %)
- eau : 1-10 % (de préférence 4-6 %)
- huile(s) : 55-95 % (de préférence 75-85 %)
- et facultativement : alcool soluble dans l'eau : 1-10 % (de préférence 2-6 %).

Pour préparer ces compositions, la phase huileuse contenant les copolymères peut être chauffée avant son introduction dans l'agent amphiphile non ionique, de préférence hydraté et contenant éventuellement un alcool. On peut aussi ajouter un seul des copolymères dans l'huile avant l'introduction dans l'agent amphiphile et compléter ensuite avec le deuxième copolymère.

L'effet observé avec l'agent amphiphile non ionique, en présence du mélange de copolymères épaississants, est surprenant car, en l'absence desdits copolymères, l'agent amphiphile à la concentration à laquelle il est utilisé, ne permet pas une gélification de l'huile : il y a décantation et recristallisation de

l'agent amphiphile. De même l'effet rhéologique de l'agent amphiphile non ionique n'est pas observé lorsqu'il est utilisé en présence d'un autre système épaississant des huiles, tel que la palmitate de dextrine ou les sels d'acides gras et d'aluminium.

Les huiles utilisées seules ou en mélanges dans les compositions obtenues selon l'invention peuvent être notamment :

- des hydrocarbures, y compris des huiles minérales, tels que les huiles de paraffine, les huiles de vaseline, le polyisobutylène hydrogéné tel que celui vendu par la Société NIPPON OIL sous la marque "Parleam", les hydrocarbures ramifiés tels que ceux vendus sous la dénomination "ISOPAR" ;
- les triglycérides, en particulier les huiles végétales, telles que l'huile de tournesol, de sésame, de colza, d'amande douce, de calophyllum, de palme, d'avocat, de jojoba, d'olive, de ricin, ou les huiles de germes de céréales comme l'huile de germes de blé ;
- divers esters huileux dérivés d'acide et/ou d'alcool à longue chaîne, tels que l'huile de Purcelin, le myristate d'isopropyle, de butyle ou de cétyle, le palmitate d'isopropyle, de butyle ou d'éthyl-2 hexyle, le stéarate d'isopropyle, de butyle, d'octyle, d'hexadécyle ou d'isocétyle, l'oléate de décyle, le laurate d'hexyle, le dicaprylate de propylèneglycol, l'adipate de di-isopropyle et les mélanges d'esters benzoïques en $C_{12}$ et $C_{15}$ vendus sous la dénomination "FINSOLV TN" par la Société WITCO, etc ;
- les huiles animales comme le perhydrosqualène ;
- les huiles de silicone comme les diméthylpolysiloxanes, les phényldiméthicones, les cyclométhicones, les alkyldiméthicones, etc ...
- les alcools à longue chaîne tels que les alcools oléique, linoléique, linolénique et isostéarylique, ou l'octyl-dodécanol ;
- les esters dérivés d'acide lanolique tels que le lanolate d'isopropyle ou d'isocétyle ;
- les acétylglycérides, les octanoates et décanoates d'alcools ou de polyalcools (notamment de glycol ou de glycérol) et les ricinoléates d'alcools ou de polyalcools, par exemple le ricinoléate de cétyle.

On peut incorporer aux huiles diverses substances lipophiles dites actives, bénéfiques pour la peau, telles que la tocophérol et ses esters, les esters gras d'acide ascorbique, l'acide 18-bêtaglycyrrhétinique, les céramides, les substances filtres absorbant l'ultraviolet, les antioxydants, etc...

L'une des caractéristiques des copolymères épaississants utilisés selon l'invention est qu'ils sont solubles dans les huiles généralement utilisées dans les compositions cosmétiques, à l'exception de certaines huiles de silicone utilisées seules.

De préférence, les huiles de silicone et les huiles végétales riches en triglycérides sont utilisées en mélange avec au moins 10 % d'une autre huile (notamment une huile minérale ou un ester d'acide gras ou d'alcool gras).

L'invention a également pour objet une composition cosmétique comportant une phase huileuse épaissie grâce à l'association de deux copolymères tels que définis précédemment.

L'invention a aussi pour objet une composition ainsi épaissie contenant en outre un agent correcteur de rhéologie tel que défini ci-dessus.

Les compositions selon l'invention constituent notamment des compositions anhydres (huiles, sticks ou gels anhydres), des huiles gélifiées, ou encore des émulsions eau-dans-l'huile ou huile-dans-l'eau.

Les compositions de l'invention constituent par exemple des huiles démaquillantes, des rouges à lèvres, des mascaras anhydres, des gels ou huiles parfumés, des huiles capillaires traitantes (anti-chute des cheveux, antipelliculaires, défrisantes, etc...), des gels ou huiles pré-bronzants, des gels ou huiles solaires, des sticks solaires, des sticks déodorants, des gels déodorants huileux, des gels huileux aromatiques pour soins de la bouche (avec ou sans bactéricides), des huiles moussantes pour les cheveux ou pour le bain, des fonds de teint.

Les exemples suivants illustrent l'invention sans toutefois la limiter.

EXEMPLES DE PREPARATION

EXEMPLE 1 : Synthèse de copolymères par polymérisation en solution Méthode générale : Dans un réacteur de 500 ml avec agitation mécanique centrale, thermomètre, réfrigérant et introduction d'azote, on ajoute successivement les monomères, le solvant ou le mélange de solvants et enfin, l'amorceur de polymérisation. On agite à température ambiante pour obtenir une solution homogène. On fait barboter de l'azote dans la réaction et on commence à chauffer le milieu jusqu'à la température réactionnelle désirée ; la montée en température est faite en 30 min. On maintient ensuite l'agitation, l'introduction d'azote et la température réactionnelle choisie pendant 10 heures. Le milieu réactionnel est ensuite ramené à la température ambiante. On purifie le polymère par précipitation dans un non solvant du produit formé mais

bon solvant des restes de monomères n'ayant pas réagi.

Séchage sous vide en étuve à température inférieure ou égale à 80 °C jusqu'à poids constant.

Les monomères utilisés sont désignés par les abréviations suivantes :

ALAU : acrylate de lauryle
AMA : acide méthacrylique
MAS : méthacrylate de stéaryle
AA : acide acrylique
MADAME : méthacrylate de diméthylaminoéthyle
ADAE : acrylate de diéthylaminoéthyle
NVP : N-vinyl pyrrolidone
AM : acrylamide
MAEH : méthacrylate d'éthyle hexyle
NDAM : N-dodécylacrylamide
NTBA : N-t-butyl acrylamide
NTOA : N-t-octyl acrylamide
ANIT : anhydride itaconique
DAAM : diacétone acrylamide

L' amorceur utilisé est l'azo bis-isobutyronitrile à des concentrations de 0,5 à 0,7 %.

Les solvants utilisés sont des mélanges de toluène et d'éthanol sauf à l'exemple 13 (tétrahydrofuranne).

L'agent précipitant est l'éthanol, sauf aux exemples 10 à 12, le méthanol aux exemples 10 et 12 ; et un mélange 50:50 de méthanol et d'eau à l'exemple 11.

La température réactionnelle est de 60 °C (exemples 1-3) ou de 65 °C (exemples 4-14).

Les résultats sont résumés dans le tableau I suivant.

L'indication T:x % signifie que l'on a utilisé comme solvant un mélange toluène-éthanol contenant x % de toluène, et donc (100 - x) % d'éthanol.

TABLEAU I

| Exemples n° | Monomères | | Solvant | Concentration des monomères | Rendement |
|---|---|---|---|---|---|
| 1 | MAS<br>AMA | 95 %<br>5 % | T = 74 % | 57 % | 97 % |
| 2 | MAS<br>MADAME | 95 %<br>5 % | T = 74 % | 57 % | 90 % |
| 3 | ALAU<br>AMA | 95 %<br>5 % | T = 74 % | 57 % | 90 % |
| 4 | MAS<br>AA | 93 %<br>7 % | T = 74 % | 59 % | 87 % |
| 5 | MAS<br>AA | 96 %<br>4 % | T = 74 % | 59 % | 89 % |
| 6 | MAS<br>ADAE | 83 %<br>17 % | T = 86 % | 59 % | 83 % |
| 7 | MAS<br>NVP | 86 %<br>14 % | T = 86 % | 59 % | 84 % |
| 8 | MAS<br>AM | 96,5 %<br>3,5 % | T = 76,5 % | 54 % | 92 % |
| 9 | MAEH<br>MADAME | 93,7 %<br>6,3 % | T = 85 % | 55,5 % | 90 % |
| 10 | NDAM<br>MADAME | 94 %<br>6 % | T = 85 % | 55,5 % | 74 % |
| 11 | NTBA<br>MADAME | 94 %<br>6 % | T = 65 % | 42 % | 84 % |
| 12 | NTOA<br>MADAME | 94 %<br>6 % | T = 58 % | 39 % | 92 % |
| 13 | MAS<br>ANIT | 94,4 %<br>5,6 % | THF | 59 % | 90 % |
| 14 | MAS<br>DAAM | 75 %<br>25 % | T = 73 % | 57 % | 91 % |

EXEMPLE 15 : Polymérisation en suspension

Dans un réacteur, on introduit 250 g d'eau distillée, 2,5 g d'hydroxyéthylcellulose et 0,13 g de mercaptoéthanol. On effectue la dissolution sous agitation et barbotage d'azote et on chauffe à 80°C. On introduit alors sous agitation 95 g de MAS préchauffé à 40°C et 5 g d'AMA.

Au bout de 5 minutes, on ajoute 0,5 ml de t-butylperoxy-2 éthylhexanoate.

Au bout de 8 heures à 80°C, on ramène à température ambiante en refroidissant sous agitation. On filtre et lave les perles obtenues avec deux fois 1 l d'eau contenant 0,4 % de laurylsulfate de sodium puis à l'eau distillée et on sèche.

Rendement : 90 %.

EXEMPLE 16 : Polymérisation en suspension

Dans un réacteur, on introduit 250 g d'eau distillée, 50 g de chlorure de sodium, 4 g d'hydroxyéthylcellulose et 0,16 g de mercaptoéthanol. On dissout sous agitation et barbotage d'azote. On chauffe à 80°C. On introduit alors 90,8 g de MAS préchauffé à 40°C et 9,2 g de MADAME. Au bout de 5 minutes d'agitation, on ajoute 0,5 ml de t-butylperoxy-2 éthylhexanoate. On termine comme indiqué à l'exemple

précédent.

Rendement : 78 %.

EXEMPLE 17 : Polymérisation en émulsion

Dans un réacteur, on introduit 298 g d'eau distillée, 4 g d'hydroxyéthylcellulose. On dissout sous agitation et barbotage d'azote, on chauffe à 50 °C. On introduit alors 46 g de stéarate de vinyle, 5,8 g d'acide crotonique et 2,5 g de peroxyde de lauroyle. On chauffe à 75 °C pendant 15 heures. On ramène ensuite à température ambiante, on filtre les perles et on les lave deux fois dans un litre d'eau. Le polymère après séchage est obtenu avec un rendement de 86%.

EXEMPLES 18 à 20 :

On a préparé de façon analogue, par polymérisation en solution, les copolymères PA1, et PA2 dont la composition est donnée aux exemples F24-F27 ci après.

EXEMPLES DE FORMULATION

Pour chacun des exemples de formulation décrite ci-dessous, on procède de la façon suivante : on dissout au préalable l'un des polymères dans une partie du mélange d'huiles considéré, en chauffant s'il le faut pour accélérer la dissolution.

L'autre polymère est dissous de la même façon dans une autre partie du mélange d'huiles. On mélange les deux solutions obtenues pour obtenir un milieu épaissi. On ajoute ensuite les autres ingrédients éventuels.

EXEMPLE F1 : Huile moussante capillaire épaissie

Cette composition a la formulation suivante :

| | |
|---|---|
| - Polymère selon l'exemple 15 | 1,0 g |
| - Polymère selon l'exemple 16 | 1,0 g |
| - Mélange lauryléther sulfate de monoisopropanol amine/diéthanolamide d'acide de coprah (50/50) vendu sous la dénomination de TEXAPON WW99 par la Société HENKEL | 35,0 g |
| - Huile de vaseline légère HZ N°25 vendue par la Société GEERAERT MATTHYS | 25,0 g |
| - Tertiobutyl 4-Hydroxyanisole | 0,05 g |
| - Ditertiobutyl 4-Hydroxytoluène | 0,05 g |
| - Parahydroxybenzoate de propyle | 0,2 g |
| - Huile de colza raffinée désodorisée qsp | 100 g |
| Viscosité de la composition 460 cp (0,46 Pa.s) | |

Cette huile épaissie s'applique sur cheveux mouillés et sales. Elle possède un bon pouvoir moussant et détergent et permet d'obtenir un bon démêlage des cheveux mouillés et une facilité de mise en forme de ces cheveux.

EXEMPLE F2 : Soin pour cheveux de type africain

Cette composition a la formulation suivante :

| | |
|---|---|
| - Polymère selon l'exemple 15 | 1,25 g |
| - Polymère selon l'exemple 16 | 1,25 g |
| - Huile de vaseline légère HZ N°25 vendue par la Société GEERAERT MATTHYS | 57,5 g |
| - Cyclopentadiméthylsiloxane vendue sous la dénomination de SILBIONE HUILE 700 45 V5 par la Société RHONE POULENC C | 40,0 g |
| Viscosité de la composition : 650 cp (0,65 Pa.s) | |

Ce produit se présente sous forme d'un gel transparent et s'applique sur cheveux mouillés, facilite le démêlage et apporte douceur et brillance aux cheveux séchés.

EXEMPLE F3 : Huile protectrice capillaire

Cette composition a la formulation suivante :

| | |
|---|---|
| - Polymère selon l'exemple 15 | 1,0 g |
| - Polymère selon l'exemple 16 | 1,0 g |
| - Hydrocarbures isoparaffiniques vendu sous la dénomination d'ISOPARH par la Société EXXON | 57,0 g |
| - Cyclopentadiméthylsiloxane vendue sous la dénomination de SILBIONE HUILE 700 45 V5 par la Société RHONE POULENC | 40,0 g |
| - 2-Hydroxy 4-méthoxy benzophénone vendue sous la dénomination d'UVINUL M40 par la Société BASF | 1,0 g |
| Viscosité de la composition : 3100 cp (3,1 Pa.s) | |

Cette huile à l'aspect gélifié s'applique sur cheveux humides. Après rinçage, les cheveux sont doux et lisses.

EXEMPLE F4 : Huile moussante parfumée pour le bain

Cette composition a la formulation suivante :

| | |
|---|---|
| - Polymère selon l'exemple 15 | 1,5 g |
| - Polymère selon l'exemple 16 | 1,5 g |
| - Mélange lauryléther sulfate de monoisopropanol amine/diéthanolamide d'acide de coprah (50/50) vendu sous la dénomination de TEXAPON WW99 par la Société HENKEL | 40,0 g |
| - Monolaurate de sorbitane oxyéthyléné à 20 moles d'oxyde d'éthylène vendu sous la dénomination de TWEEN 20 par la Société ICI | 5,0 g |
| - Huile de sésame vierge | 25,0 g |
| - Ditertiobutyl-4 Hydroxytoluène | 0,1 g |
| - Tertiobutyl-4 Hydroxyanisole | 0,04 g |
| - Parahydroxybenzoate de propyle | 0,15 g |
| - Parfum | 1,0 g |
| - Huile de colza raffinée désodorisée qsp | 100 g |

Cette huile de bain s'utilise en baignoire à raison de 10 g pour 100 l d'eau.

Elle confère en final à la peau douceur et aspect satiné.

EXEMPLE F5 : Fond de teint (émulsion E/H)

Cette composition a la formulation suivante :

| | |
|---|---|
| - Polymère selon l'exemple 15 | 0,15 g |
| - Polymère selon l'exemple 16 | 0,15 g |
| - Myristate d'isopropyle | 11,00 g |
| - Petrolatum | 4,00 g |
| - P.E.G. 7 Hydrogenated castor oil | 3,00 g |
| - Cire d'abeilles | 7,50 g |
| - Paraben | 0,12 g |
| - Tertiobutyl-4-hydroxy anisole (BHA) | 0,15 g |
| - Oxyde de fer | 1,50 g |
| - Oxyde de titane | 11,50 g |
| - Octyldiméthyl PABA* | 1,00 g |
| - Cyclomethicone | 9,50 g |
| - Eau | 38,35 g |
| - Propylène glycol | 4,00 g |
| - Diazolidinylurée | 0,20 g |
| - Sulfate de magnésium | 0,70 g |
| - Amidon de maïs | 7,00 g |
| - Alcool phényléthylique | 0,18 g |
| TOTAL | 100 g |

* PABA : Paradiméthylaminobenzoate d'éthyl-2 hexyle.

EXEMPLE F6 : Fond de teint

Même formule qu'à l'exemple 5 avec le couple de polymères.

| | |
|---|---|
| - Polymère selon l'exemple 3 | 0,15 g |
| - Polymère selon l'exemple 16 | 0,15 g |

EXEMPLE F7 : Rouge à lèvres

| | |
|---|---|
| - Polymère selon l'exemple 3 | 0,30 g |
| - Polymère selon l'exemple 16 | 0,30 g |
| - Octylhydroxystéarate | 13,20 g |
| - Octylstéarate | 6,60 g |
| - Huile de ricin | 6,60 g |
| - Huile de vaseline | 13,20 g |
| - 3,5 Di-tertiobutyl-4-hydroxy toluène (BHT) | 0,10 g |
| - Lanoline | 22,60 g |
| - Vinylacétate/allyl stéarate copolymère | 11,30 g |
| - Cire de carnauba | 2,80 g |
| - Cire microcristalline | 11,30 g |
| - F D et C Yellow n°6 | 7,30 g |
| - D et C Red n°7 | 2,00 g |
| - Oxyde de fer | 1,50 g |
| - Oxyde de titane | 0,65 g |
| - Parfum | 0,25 g |
| TOTAL | 100 g |

EXEMPLE F8 : Mascara

| | |
|---|---|
| - Cire de paraffine | 27,7 g |
| - Acide stéarique | 3,8 g |
| - Amidon | 1,3 g |
| - Oxyde de fer | 6,3 g |
| - Isoparaffine | 59,6 g |
| - Polymère selon l'exemple 16 | 0,65 g |
| - Polymère selon l'exemple 15 | 0,65 g |

EXEMPLE F9 : Huile démaquillante

| | | |
|---|---|---|
| Solution A : | Isohexadécane<br>Copolymère acrylate de Lauryle/acide méthacrylique selon l'exemple 3. | 48,75 g<br>1,5 g |
| Solution B : | Isohexadecane<br>Méthacrylate de stéaryle/M.A.D.A.M.E selon l'exemple 16. | 48,75 g<br>1 g |

Mode opératoire :

Les solutions A et B sont préparées à 80 °C sous agitation magnétique.

- Quand A et B sont limpides, les mélanger doucement à 80 °C sous agitation magnétique. Laisser agiter à cette température pendant environ 2 heures.
- Arrêter l'agitation et laisser refroidir à température ambiante. On obtient un gel huileux transparent.

EXEMPLE F10 : Formule solaire

| | |
|---|---|
| - Polymère selon l'exemple 3 | 1,25 g |
| - Polymère selon l'exemple 16 | 1,25 g |
| - 3',5' ditertiobutyl-4'hydroxy-3 benzylidène camphre | 1,5 g |
| - Finsolv TN qsp | 100 g |

EXEMPLE F11 : Formule après-soleil

| | |
|---|---|
| - Polymère selon l'exemple 16 | 1 g |
| - Polymère selon l'exemple 3 | 1,5 g |
| - Bisabolol | 0,5 g |
| - Huile de vaseline qsp | 100 g |

EXEMPLE F12 : Formule solaire avec filtre UVA

| | |
|---|---|
| - Polymère selon l'exemple 16 | 1,5 g |
| - Polymère selon l'exemple 15 | 1 g |
| - Parsol 1789 (Tertiobutyl méthoxydibenzoylmethane) commercialisé par GIVAUDAN | 1,25 g |
| - Myristate d'isopropyle | 20 g |
| - Huile de vaseline qsp | 100 g |

EXEMPLE F13 : Formule solaire avec filtre UVB

| | |
|---|---|
| - Polymère selon l'exemple 3 | 1,125 g |
| - Polymère selon l'exemple 16 | 0,75 g |
| - Witiconol APM * | 24 g |
| - Uvinul T150 (2,4,6 trianilinoparacarbo-2'-éthylhexyl-1'-oxy-1,3,5-triazine) | 1 g |
| - Parleam ** qsp | 100 g |

*(Polypropylène glycol éther de l'alcool myristique) commercialisé par WITCO ORGANICS,
**(Polyisobutène hydrogéné) commercialisé par NIPPON OIL.

EXEMPLE F14 : Formule solaire avec polymère filtre

| | |
|---|---|
| - Polymère selon l'exemple 16 | 1,25 g |
| - Polymère selon l'exemple 3 | 1,25 g |
| - Homopolymère dérivé de polyacrylamide contenant des motifs de formule I | 1,25 g |
| - Ester palmitique du 2-éthyl hexyl glycérol éther qsp | 100 g |

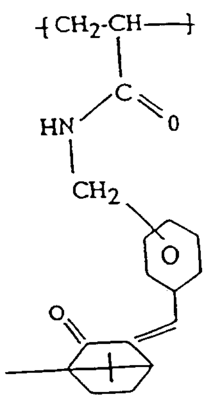

FORMULE I

EXEMPLE F15 : Formule solaire avec polymère filtre

| | |
|---|---|
| - Polymère selon l'exemple 16 | 1,5 g |
| - Polymère selon l'exemple 15 | 1,5 g |
| - Homopolymère dérivé de polyacrylamide contenant des motifs de formule II | 1,5 g |
| - Myristate d'isopropyle qsp | 100 g |

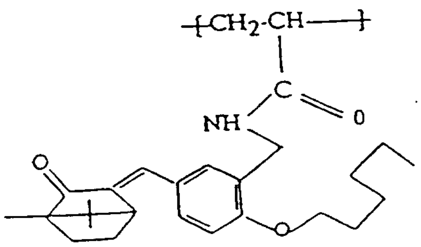

FORMULE II

EXEMPLE F16 : Huile antimoustique

| | |
|---|---|
| - Polymère selon l'exemple 15 | 1,0 g |
| - Polymère selon l'exemple 16 | 1,0 g |
| - Huile essentielle de citronnelle | 10,0 g |
| - Silicone volatile 700 45 V5 (Rhône Poulenc) commercialisé sous le nom de CYCLOMETHICONE | 40,0 g |
| - Huile de vaseline qsp | 100 g |

EXEMPLE F17 : Gel anti-inflammatoire contenant un corticoïde en milieu lipophile

| | |
|---|---|
| - Parleam | 67,78 g |
| - Myristate d'isopropyle | 30 g |
| - Polymère selon l'exemple 15 | 1 g |
| - Polymère selon l'exemple 16 | 1 g |
| - 17 butyrate d'hydrocortisone | 0,2 g |
| - BHA | 0,002 g |

EXEMPLE F18 : Gel pour le traitement du psoriasis contenant du dithranol

| | |
|---|---|
| - Huile de vaseline fluide | 60,4 g |
| - Cyclométhicone | 35 g |
| - Polymère selon l'exemple 3 | 1,75 g |
| - Polymère selon l'exemple 16 | 1,75 g |
| - Dithranol | 1 g |
| - BHA | 0,05 g |
| - BHT | 0,05 g |

Formule F19 : Formule solaire

| | |
|---|---|
| - Polymère selon l'exemple 3 | 0,5 g |
| - Polymère selon l'exemple 16 | 0,5 g |
| - 3',5' ditertiobutyl-4' hydroxy-3 benzylidène camphre | 0,5 g |
| - Sinnowax AO | 3 g |
| - Mélange mono et distéarate de glycérol non auto émulsionnable | 1 g |
| - Alcool cétylique | 1 g |
| - Huile silicone 700 47V 300 | 1 g |
| - Finsolv TN | 10 g |
| - Glycérine | 20 g |
| - Conservateur qs | |
| - Eau purifiée qsp | 100 g |

EXEMPLE F20 : Formule après-soleil

| | |
|---|---|
| - Polymère selon l'exemple 16 | 0,75 g |
| - Polymère selon l'exemple 15 | 0,75 g |
| - Bisabolol | 0,5 g |
| - Sinnowax AO | 3 g |
| - Mélange mono et distéarate de glycérol non auto émulsionnable | 1 g |
| - Alcool cétylique | 1 g |
| - Huile de vaseline | 9 g |
| - Glycérine | 20 g |
| - Conservateur qs | |
| - Eau purifiée qsp | 100 g |

EXEMPLE F21 : Formule antisolaire

| | |
|---|---|
| - Oxyde de titane enrobé d'alumine et de stéarate d'aluminium vendu sous la dénomination MICRO $TIO_2$ MT 100T par la Société TAYCA | 5 g |
| - Polymère selon l'exemple 16 | 0,4 g |
| - Polymère selon l'exemple 3 | 0,4 g |
| - Arlacel 780 | 5 g |
| - Huile de vaseline | 14 g |
| - Finsolv TN | 6 g |
| - Glycérine | 4 g |
| - Sulfate de magnésium | 0,7 g |
| - Conservateur qs | |
| - Eau purifiée qsp | 100 g |

EXEMPLE F22 : Formule antisolaire

| | |
|---|---|
| - Uvinul T150 | 1 g |
| - Polymère selon l'exemple 16 | 0,6 g |
| - Polymère selon l'exemple 15 | 0,6 g |
| - Arlacel 780 | 5 g |
| - Witconol APM | 20 g |
| - Glycérine | 4 g |
| - Sulfate de magnésium | 0,7 g |
| - Conservateur qs | |
| - Eau purifiée qsp | 100 g |

EXEMPLE F23 : Formule antisolaire

| | |
|---|---|
| - Parsol 1789 | 1,5 g |
| - Polymère selon l'exemple 16 | 1 g |
| - Polymère selon l'exemple 3 | 1 g |
| - Myristate d'isopropyle | 20 g |
| - Conservateurs qs | |
| - Arlacel 780 | 5 g |
| - Eau purifiée qsp | 100 g |

EXEMPLE F 24 à F27 : Préparations gélifiées

PA1 : polymère obtenu à partir des monomères : méthacrylate de stéaryle, acide méthacrylique, acrylate de lauryle (56,6: 3,4:40).
PA2 : polymère obtenu à partir des monomères : acrylate de lauryle, acide méthacrylique (96,6:3,4)

A l'aide de ces copolymères on a obtenu les préparations gélifiées (F24 à F27) suivantes :

EXEMPLE F :

| | 24 | 25 | 26 | 27 |
|---|---|---|---|---|
| PA1 | 1,165 | 1,75 | 1,165 | 0,6 |
| PA2 | 1,165 | 1,75 | 1,165 | 1,2 |
| Polymères de l'exemple 2 | 2,34 | 3,5 | 2,34 | 1,8 |
| Huile de vaseline | 72 | 69,5 | 38,17 | 28,6 |
| Cetearyl octanoate et isopropul myristate (90/10) (DUB liquide 85 IP des STEARINERIES de DUBOIS) | 0 | 0 | 33,66 | 0 |
| Volatil silicone (D.C. Fluid 245) | 0 | 0 | 0 | 25 |
| Triglycéride caprylique/caprique (Mygliol 812 de HULS) | 0 | 0 | 0 | 26,3 |
| Octyl 2 dodécyléther oxyéthyléné (25OE) (Emalex OD25 de NIHON EMULSION) | 9,33 | 9,4 | 9,4 | 7 |
| Eau | 9,33 | 9,4 | 9,4 | 6 |
| Glycérol | 4,67 | 4,7 | 4,7 | 3,5 |

Ces gels (F24 à F27) sont souples à la prise et s'étalent bien. On les utilise de préférence dans des émulsions pour épaissir et stabiliser celles-ci comme indiqué ci-dessous.

EXEMPLE 28 : Emulsion E/H

| | | |
|---|---|---|
| A | Lanolate de magnésium - huile de vaseline (50/50) (Mexanyl GO de CHIMEX) | 5,7 |
| | Lanoline hydrogénée (SUPERSAT de RITA) | 6,65 |
| | Palmitate d'éthyl-2 hexyle glycéryl éther (octoxyglycéryl palmitate) | 2 |
| | Alcool de lanoline - huile de vaseline (15/85) (AMERCHOL L 101 de AMERCHOL) | 3 |
| | Huile de vaseline | 9,25 |
| | Ceterayl octanoate - isopropyle myristate (90/10)) (DUB LIQUIDE 85 IP des STEARINERIES de DUBOIS) | 7,95 |
| B | Eau | 50,45 |
| C | Préparation gélifiée F 24 | 15 |

On prépare la phase grasse A, on y ajoute l'eau (phase B) et lorsque l'émulsion est formée, on y introduit la phase C à une température inférieure à 40 °C sous faible agitation. On obtient une crème blanche agréable à étaler.

EXEMPLE F 29 : EMULSION

| | | |
|---|---|---|
| A | Isostéarate de sorbitane (Arlacel 987) de ICI) | 5 |
| | Huile de vaseline | 15,4 |
| | Propylène glycol | 3 |
| B | Sulfate de magnésium | 0,6 |
| | Eau | 65 |
| C | Préparation gélifiée F25 | 11 |

On prépare la phase grasse A, on y ajoute la phase aqueuse B, et après obtention de l'émulsion, on y introduit la phase C à une température inférieure à 40 °C sous faible agitation. On obtient une crème blanche agréable à l'application et qui pénètre bien.

EXEMPLE F 30 : Emulsion E/H:

| | | |
|---|---|---|
| A | Mono-diglycéryl d'isostéaryl succinate (Inwitor 780K de HULS) | 5 |
| | Huile de vaseline | 11,2 |
| | Caprylique-caprique triglycéride (Mygliol 812 de HULS) | 9,3 |
| | Volatil silicone (D.C. Fluid 245) | 4,5 |
| B | Eau | 48 |
| | Sulfate de magnésium | 2 |
| | Glycérol | 3 |
| | Amidon de maïs | 7 |
| C | Préparation gélifiée F 27 | 10 |

On prépare cette crème comme indiqué dans les exemples précédents. On obtient une crème blanche et onctueuse, agréable à utiliser.

EXEMPLE F 31 : EMULSION E/H :

| | | |
|---|---|---|
| A | Mono-diglycéryl d'isostéaryl succinate (Inwitor 780K de HULS) | 5 |
| | Polyphényl méthyl siloxane (Silbione 70633V30-RHONE-POULENC | 7 |
| | Caprylique-caprique triglycéride (Mygliol 812 de HULS) | 10 |
| | Volatil silicone (D.C. Fluid 245) | 8 |
| B | Eau | 45 |
| | Sulfate de magnésium | 2 |
| | Glycérol | 3 |
| | Amidon de maïs | 10 |
| C | Préparation gélifiée F26 | 10 |

On prépare cette crème comme indiqué dans les exemples précédents. On obtient une crème blanche, onctueuse, fraîche à l'application.

EXEMPLE F32 à F34 : GELS DEMAQUILLANTS RINCABLES A L'EAU

EXEMPLE F :

| | | 32 | 33 | 34 |
|---|---|---|---|---|
| A | PA1 | 0,25 | 1,5 | 0,25 |
| | PA2 | 0,75 | 0 | 0,75 |
| | Polymères de l'exemple 2 | 1 | 1,5 | 1 |
| | Huile de vaseline | 2 | 3 | 2 |
| | Huile de jojoba | 78 | 78 | 71 |
| | Silicone volatile (D.C. 245) | 0 | 0 | 5 |
| B | Octyldodécyléther oxyéthyléné (25 OE) (EMALEX OD 25 de NIHON EMULSION) | 5 | 4 | 5 |
| | Eau | 5 | 4 | 5 |
| | Glycérol | 4 | 4 | 5 |
| | Oléate de sorbitan oxyéthyléné (40 OE) (ARLATONE T de ICI) | 4 | 4 | 4 |
| | Décyléther de glucoside | 0 | 0 | 1 |
| | (ORAMIX NS 10 de SEPPIC) | 0 | 0 | 1 |

On prépare ces gels par introduction lente de la phase A chauffée à 60° dans la phase B fondue. On agite fortement jusqu'à l'obtention du gel qui est rapidement refroidi sous faible agitation.

Les gels obtenus, riches en huile de jojoba, sont particulièrement confortables à utiliser. Lors de l'utilisation, on les applique sur peau sèche ou humide, on masse légèrement pour résorber le maquillage puis on rince à l'eau.

EXEMPLES F 35 : GEL DEMAQUILLANT :

| | | |
|---|---|---|
| A | PA1 .................................... | 0,25 |
| | PA2 .................................... | 0,75 |
| | Polymères de l'exemple 2 ............... | 1 |
| | Huile de vaseline ...................... | 2 |
| | Huile de jojoba | 72 |
| | Silicone volatil (D.C. 245) ............ | 5 |
| B | Octyldodécyléther oxyéthyléné (20 OE) (EMALEX OD 20 de NIHON EMULSION) | 5 |
| | Eau .................................... | 5 |
| | Glycérol ............................... | 5 |
| | Oléate de sorbitan oxyéthyléné (40 OE) (ARLATONE T de ICI) | 4 |

Le mode opératoire est le même que pour les exemples précédents. Le gel obtenu a les mêmes propriétés.

EXEMPLE F36 à F39 : GELS DEMAQUILLANTS :

EXEMPLE F :

| | | 36 | 37 | 38 | 39 |
|---|---|---|---|---|---|
| A | PA1 | 0,4 | 0,07 | 0,25 | 0,25 |
| | PA2 | 1,25 | 0,215 | 0,75 | 0,75 |
| | Polymères de l'exemple 2 | 1,65 | 0,285 | 1 | 1 |
| | Huile de vaseline | 3,3 | 72 | 7 | 35,26 |
| | Huile de jojoba | - | 15,15 | 10 | 1 |
| | Isoparaffine hydrogénée (Parleam de NIPPON OIL) | 37,7 | - | - | - |
| | Palmitate d'éthyl-2 hexyle (Céraphyl 368 de MALLINCKRODT) | 37,7 | 5,7 | 60 | 30,5 |
| | Stéaryl octanoate et isopropyl myristate (90/10)(DUB liquide 85 IP des STEARINERIES DUBOIS) | - | - | - | - |
| | Silicone volatile (D.C. Fluid 245) | - | - | - | 10 |
| | Parfum | - | - | - | 0,2 |
| B | Octyldodécyléther oxyéthyléné (25 OE) (EMALEX OD 25 de NIHON EMULSION) | 4,8 | 1,43 | 5 | 4,5 |
| | Eau | 5,7 | 1,14 | 5 | 4,5 |
| | Glycérol | 3,8 | 1,71 | 6 | 6 |
| | Butylène 1,3 glycol | - | 1,14 | - | - |
| | Oléate de sorbitan oxyéthyléné (40 OE) (ARLATONE T de ICI) | 3,8 | 1,14 | 4 | 4 |
| | Antioxydant | - | 0,01 | - | 0,04 |

Ces gels sont préparés selon le mode opératoire décrit précédemment.

On obtient des gels de démaquillage particulièrement confortables à utiliser, permettant rapidité et efficacité du démaquillage. Ces gels, testés sur les utilisatrices, ont été appréciés favorablement.

A titre d'exemple, le gel F39 a été jugé sur un panel de 16 femmes qui l'ont trouvé facile et rapide à appliquer, qui ont jugé son pouvoir démaquillant très bon et qui ont constaté une impression de confort après démaquillage (absence de film gras, peau douce et nette).

EXEMPLE F40 : MASQUE HYDRATANT RINCABLE

| | | |
|---|---|---|
| A | PA1 | 0,5 |
| | PA2 | 1,5 |
| | Polymères de l'exemple 2 | 2 |
| | Huile de vaseline | 25 |
| | Huile d'amande d'abricot | 10 |
| | Silicone volatil (D.C. FLUID 245) | 20 |
| B | Eastman AQ 55 S polymer (Polyester, Polyisophtalate) | 2 |
| | Octyldodécyléther oxyéthyléné (25 OE) (EMALEX OD 25 de NIHON EMULSION) | 7 |
| | Eau | 7 |
| | Glycérol | 24 |
| | Oléate de sorbitan oxyéthyléné (40 OE) (ARLATONE T de ICI) | 1 |

On prépare ce masque selon le mode opératoire décrit ci-dessus pour les gels démaquillants.

On obtient un gel translucide que l'on applique en masque sur le visage. Après 5 à 10 minutes d'application, on rince à l'eau

EXEMPLE F 41 : GEL POUR LE CORPS A BASE D'HUILES

Préparation gélifiée pour peaux sèches riche en huiles émolientes, pénétrant bien et ne laissant pas de film gras sur la peau. Cette composition présente l'avantage de ne pas devoir être rincée après application, tout en contenant un fort taux d'huile.

EXEMPLE F 42 : GEL CAPILLAIRE A RINCER APRES APPLICATION.

Ce gel nourrit et gaine le cheveu et améliore le coiffage.

Les formulations sont les suivantes :

EXEMPLE F

| | 41 | 42 |
|---|---|---|
| PA1 | 0,25 | 0,5 |
| PA2 | 0,75 | 2 |
| Polymères suivant Exemple 2 | 1 | 2,5 |
| triglycérides d'acides caprique et caprylique (Miglyol 812 - HULS FRANCE) | 7 | - |
| Dicaprylate de propylène glycol (Crodamol PC - Croda) | 20 | - |
| Néopentanoate d'isostéaryle (Céraphyl 375 - Mallinckrodt) | 6 | - |
| Squalane (Johan Martens) | 6 | 9 |
| Diisopropyl dimerdilinoléate (Schercemol DID-SCHER) | | 15 |
| Volatil silicone (D4) (Dow Corning Fluid 244) | 30 | - |
| Volatil silicone (D5) (Dow Corning Fluid 245) | 20 | 48 |
| Gomme de silicone (Dow Corning QC F2 - 1671) | - | 3 |
| Octyl dodécyl éther d'oxyde d'éthylène (25 OE) (EMALEX-OD-25 de NIHON EMULSION) | 2 | 4 |
| Eau | 3 | 5 |
| Glycérol | 4 | 7 |
| Oléate de sorbitan oxyéthyléné (40 OE) (ARLATONE T ICI) | - | 4 |

**Revendications**

1. Utilisation en association, comme agents épaississants des huiles, dans une composition cosmétique comprenant une phase huileuse, d'au moins un premier copolymère comprenant des motifs dérivés d'au moins un monomère lipophile et des motifs A dérivés d'au moins un monomère hydrophile comprenant au moins un groupement acide carboxylique ou sulfonique, et d'au moins un second copolymère comportant des motifs dérivés d'au moins un monomère lipophile et des motifs B dérivés d'au moins un monomère hydrophile comprenant au moins un groupement amine, amide, alcool ou éther, lesdits premier et second copolymères ayant une masse moléculaire non inférieure à 100 000.

2. Utilisation selon la revendication 1, caractérisée par le fait que dans lesdits premier et second copolymères, la proportion pondérale des motifs dérivés du monomère lipophile est au moins égale à 50 %.

3. Utilisation selon la revendication 1 ou 2, caractérisée par le fait que dans lesdits premier et second copolymères, la proportion pondérale des motifs A et des motifs B, respectivement, est au moins égale à 2 %.

4. Utilisation selon la revendication 1, caractérisée par le fait que lesdits motifs A sont dérivés d'au moins un monomère choisi parmi les acides carboxyliques insaturés et leurs dérivés et les acides sulfoniques insaturés.

5. Utilisation selon la revendication précédente, caractérisée par le fait que lesdits acides carboxyliques insaturés et leurs dérivés comprennent :
   - les monoacides carboxyliques insaturés,
   - et les diacides carboxyliques insaturés, leurs monoesters et leurs monoamides.

6. Utilisation selon la revendication précédente, caractérisée par le fait que lesdits monoacides carboxyliques insaturés sont choisis parmi l'acide acrylique, l'acide méthacrylique et l'acide crotonique, et/ou que lesdits diacides sont choisis parmi l'acide maléique et l'acide itaconique, et/ou que lesdits monoesters ou monoamides dérivent respectivement d'alcools ou d'amines ayant de 1 à 22 atomes de carbone.

7. Utilisation selon l'une quelconque des revendications 2 à 4, caractérisée par le fait que lesdits acides sulfoniques insaturés sont choisis parmi l'acide acrylamido-2 méthyl-2 propanesulfonique et le méthacrylate de 2-sulfoéthyle.

**8.** Utilisation selon l'une quelconque des revendications précédentes, caractérisée par le fait que lesdits motifs B dérivés d'au moins un monomère hydrophile comprenant un groupement amine répondent à la formule :

$$\left[ CH_2C(R_1) \right] \\ \qquad CO{-}M{-}(CH_2)_n{-}N(R_2)(R_3)$$

dans laquelle :

- M représente -O- ou -NH-,
- $R_1$ représente -H ou $-CH_3$,
- n est un nombre de 2 à 20,
- $R_2$ et $R_3$ représente indépendamment -H ou un groupement hydrocarboné ayant 1 à 4 atomes de carbone.

**9.** Utilisation selon la revendication précédente, caractérisée par le fait que lesdits motifs B sont dérivés de monomères choisis parmi le méthacrylate de diméthylaminoéthyle, l'acrylate de diéthylaminoéthyle et le N-diméthylaminopropyl méthacrylamide.

**10.** Utilisation selon l'une quelconque des revendications précédentes, caractérisée par le fait que lesdits motifs B sont dérivés d'au moins un monomère hydrophile insaturé comprenant un groupement amide, choisis parmi l'acrylamide, le méthacrylamide, le N,N-diméthylacrylamide, la N-vinyl pyrrolidone et le diacétone acrylamide.

**11.** Utilisation selon l'une quelconque des revendications précédentes, caractérisée par le fait que lesdits copolymères ont une masse moléculaire au moins égale à 200 000.

**12.** Utilisation selon l'une quelconque des revendications précédentes, caractérisée par le fait que les proportions pondérales relatives desdits premier et second copolymères dans ladite association, sont dans l'intervalle de 10:90 à 90:10 et en particulier dans l'intervalle de 25:75 à 75:25.

**13.** Utilisation selon la revendication précédente, caractérisée par le fait que la concentration totale desdits premier et second copolymères dans ladite composition est inférieure à 10 % et en particulier inférieure à 5 % en poids.

**14.** Utilisation selon la revendication précédente, caractérisée par le fait que la concentration totale desdits copolymères est supérieure à 0,1 % en poids.

**15.** Utilisation selon l'une quelconque des revendications précédentes, caractérisée par le fait que ladite phase huileuse comprend des hydrocarbures, des triglycérides, ou des esters d'acide et/ou d'alcool à longue chaîne.

**16.** Utilisation selon l'une quelconque des revendications précédentes, caractérisée par le fait que l'on dissout chaque copolymère dans une partie de l'huile, ou du mélange d'huiles, à épaissir, et que l'on mélange ensuite les deux solutions obtenues.

**17.** Utilisation selon la revendication 16, caractérisée par le fait que l'on ajoute les ingrédients oléosolubles de la composition, avant ou après addition du copolymère, à l'une et/ou à l'autre partie de l'huile, avant d'effectuer ledit mélange.

**18.** Utilisation selon l'une quelconque des revendications précédentes, caractérisée par le fait que l'on introduit en outre dans ladite phase huileuse un agent correcteur de rhéologie.

**19.** Utilisation selon la revendication précédente, caractérisée par le fait que ledit agent correcteur de rhéologie est un agent amphiphile non ionique.

**20.** Utilisation selon la revendication précédente, caractérisée par le fait que ledit agent amphiphile a une valeur de HLB comprise entre 12 et 40.

**21.** Utilisation selon l'une quelconque des revendications 18 à 20, caractérisée par le fait que ledit correcteur de rhéologie est choisi parmi :
- les esters d'acides gras et de sorbitan polyoxyéthyléné,
- les esters d'acides gras et de glycérol polyoxyéthyléné,
- les esters d'acides gras et de propylène glycol polyoxyéthyléné,
- les alkyl éthers polyoxyéthylénés ou polyoxypropylénés,
- les alkyl phényl éthers polyoxyéthylénés ou polyoxypropylénés,
- les alcools de Guerbet polyoxyéthylénés.

**22.** Utilisation selon l'une quelconque des revendications 19 à 21, caractérisée par le fait que ledit agent amphiphile est utilisé sous forme hydratée.

**23.** Utilisation selon la revendication précédente, caractérisée par le fait que ledit agent amphiphile est utilisé en présence, en outre, d'un alcool soluble dans l'eau.

**24.** Composition cosmétique caractérisée par le fait qu'elle comporte une phase huileuse épaissie telle que définie dans l'une quelconque des revendications 1 à 23.

**25.** Composition cosmétique selon la revendication précédente, caractérisée par le fait qu'elle contient en outre un agent correcteur de rhéologie.

**26.** Composition cosmétique selon la revendication précédente, caractérisée par le fait que ledit agent correcteur de rhéologie est tel que défini dans l'une quelconque des revendications 19 à 23.

**27.** Composition cosmétique selon la revendication précédente, caractérisée par le fait que ladite phase huileuse contient (% en poids total 100 % ) :
- mélange épaississant de copolymères : 0,5-10 % (de préférence 2-4 %)
- agent amphiphile non ionique : 1-10 % (de préférence 4-6 %)
- eau : 1-10 % (de préférence 4-6 %)
- huile(s) : 55-95 % (de préférence 75-85 %)
- et facultativement : alcool soluble dans l'eau : 1-10 % (de préférence 2-6 %).

**28.** Composition cosmétique selon l'une quelconque des revendications 24 à 27, caractérisée par le fait qu'elle est sous forme d'émulsion.

## Claims

**1.** Use in combination, as thickening agents for oils, in a cosmetic composition comprising an oily phase, of at least a first copolymer comprising units derived from at least one lipophilic monomer and units A derived from at least one hydrophilic monomer comprising at least one carboxylic or sulphonic acid group, and of at least a second copolymer containing units derived from at least one lipophilic monomer and units B derived from at least one hydrophilic monomer comprising at least one amine, amide, alcohol or ether group, the said first and second copolymers having a molecular weight not less than 100,000.

**2.** Use according to Claim 1, characterized in that, in the said first and second copolymers, the weight proportion of the units derived from the lipophilic monomer is at least equal to 50 %.

**3.** Use according to Claim 1 or 2, characterized in that, in the said first and second copolymers, the weight proportion of the units A and of the units B, respectively, is at least equal to 2 %.

**4.** Use according to Claim 1, characterized in that the said units A are derived from at least one monomer chosen from unsaturated carboxylic acids and derivatives thereof and unsaturated sulphonic acids.

**5.** Use according to the preceding claim, characterized in that the said unsaturated carboxylic acids and the derivatives thereof comprise:
- unsaturated carboxylic monoacids,
- and unsaturated carboxylic diacids, monoesters thereof and monoamides thereof.

**6.** Use according to the preceding claim, characterized in that the said unsaturated carboxylic monoacids are chosen from acrylic acid, methacrylic acid and crotonic acid, and/or in that the said diacids are chosen from maleic acid and itaconic acid, and/or in that the said monoesters or monoamides are derived respectively from alcohols or amines having from 1 to 22 carbon atoms.

**7.** Use according to any one of Claims 2 to 4, characterized in that the said unsaturated sulphonic acids are chosen from 2-acrylamide-2-methylpropanesulphonic acid and 2-sulphoethyl methacrylate.

**8.** Use according to any one of the preceding claims, characterized in that the said units B derived from at least one hydrophilic monomer comprise an amine group corresponding to the formula:

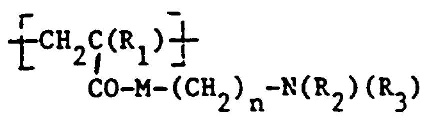

in which:
- M represents -O- or -NH-,
- $R_1$ represents -H or $-CH_3$,
- n is a number from 2 to 20,
- $R_2$ and $R_3$ independently represent -H or a hydrocarbon group having 1 to 4 carbon atoms.

**9.** Use according to the preceding claim, characterized in that the said units B are derived from monomers chosen from dimethylaminoethyl methacrylate, diethylaminoethyl acrylate and N-dimethylaminopropylmethacrylamide.

**10.** Use according to any one of the preceding claims, characterized in that the said units B are derived from at least one unsaturated hydrophilic monomer comprising an amide group, chosen from acrylamide, methacrylamide, N,N-dimethylacrylamide, N-vinylpyrrolidone and diacetone acrylamide.

**11.** Use according to any one of the preceding claims, characterized in that the said copolymers have a molecular weight at least equal to 200,000.

**12.** Use according to any one of the preceding claims, characterized in that the relative weight proportions of the said first and second copolymers in the said combination are in the range from 10:90 to 90:10 and in particular in the range from 25:75 to 75:25.

**13.** Use according to the preceding claim, characterized in that the total concentration of the said first and second copolymers in the said composition is less than 10 %, and in particular less than 5 %, by weight.

**14.** Use according to the preceding claim, characterized in that the total concentration of the said copolymers is greater than 0.1 % by weight.

**15.** Use according to any one of the preceding claims, characterized in that the said oily phase comprises hydrocarbons, triglycerides or esters of acid and/or of alcohol containing a long chain.

**16.** Use according to any one of the preceding claims, characterized in that each copolymer is dissolved in a part of the oil, or of the mixture of oils, to be thickened, and in that the two solutions obtained are then mixed together.

**17.** Use according to Claim 16, characterized in that the oil-soluble ingredients of the composition are added, before or after addition of the copolymer, to one and/or to the other part of the oil, before the said mixing is carried out.

**18.** Use according to any one of the preceding claims, characterized in that a rheology-modifying agent is also added into the said oily phase.

**19.** Use according to the preceding claim, characterized in that the said rheology-modifying agent is a nonionic amphiphilic agent.

**20.** Use according to the preceding claim, characterized in that the said amphiphilic agent has an HLB value between 12 and 40.

**21.** Use according to any one of Claims 18 to 20, characterized in that the said rheology-modifier is chosen from:

- fatty acid esters of polyoxyethylenated sorbitan,
- fatty acid esters of polyoxyethylenated glycerol,
- fatty acid esters of polyoxyethylenated propylene glycol,
- polyoxyethylenated or polyoxypropylenated alkyl ethers,
- polyoxyethylenated or polyoxypropylenated alkyl phenyl ethers,
- polyoxyethylenated Guerbet alcohols.

**22.** Use according to any one of Claims 19 to 21, characterized in that the said amphiphilic agent is used in hydrated form.

**23.** Use according to the preceding claim, characterized in that the said amphiphilic agent is used in the additional presence of a water-soluble alcohol.

**24.** Cosmetic composition, characterized in that it contains a thickened oily phase as defined in any one of Claims 1 to 23.

**25.** Cosmetic composition according to the preceding claim, characterized in that it also contains a rheology-modifying agent.

**26.** Cosmetic composition according to the preceding claim, characterized in that the said rheology-modifying agent is as defined in any one of Claims 19 to 23.

**27.** Cosmetic composition according to the preceding claim, characterized in that the said oily phase contains (% by weight, total 100 %):

- thickening mixture of copolymers: 0.5-10 % (preferably 2-4 %)
- nonionic amphiphilic agent: (1-10 % (preferably 4-6 %)
- water: 1-10 % (preferably 4-6 %)
- oil(s): 55-95 % (preferably 75-85 %)
- and optionally: water-soluble alcohol: 1-10 % (preferably 2-6 %).

**28.** Cosmetic composition according to any one of Claims 24 to 27, characterized in that it is in emulsion form.

**Patentansprüche**

**1.** Verwendung einer Kombination aus Verdickungsmitteln von Ölen in kosmetischen Zubereitungen mit einem Gehalt an einer Ölphase aus mindestens einem ersten Copolymer mit von mindestens einem lipophilen Monomeren abgeleiteten Einheiten und mit Einheiten A, welche mindestens von einem hydrophilen Monomeren mit mindestens einer Carbon- oder Sulfonsäuregruppe abgeleitet wurden, sowie mit mindestens einem zweiten Copolymeren, welches mindestens von einem lipophilen Monomeren abgeleitete Einheiten sowie Einheiten B aufweist, welche von mindestens einem hydrophilen Monomeren mit mindestens einer Amin-, Amid-, Alkohol- oder Ethergruppierung abgeleitet sind, wobei das erste und das zweite Copolymer ein Molekulargewicht von nicht weniger als 100 000 aufweisen.

**2.** Verwendung gemäß Anspruch 1, dadurch gekennzeichnet, daß das erste und zweite Copolymer ein Gewichtsverhältnis der von dem lipophilen Monomeren abgeleiteten Einheiten von mindestens oder gleich 50 % aufweisen.

**3.** Verwendung gemäß den Ansprüchen 1 oder 2, dadurch gekennzeichnet, daß das Gewichtsverhältnis bei dem ersten und dem zweiten Copolymeren in bezug auf die Einheiten A und die Einheiten B mindestens oder gleich 2 % beträgt.

**4.** Verwendung gemäß Anspruch 1, dadurch gekennzeichnet, daß die Einheiten A von mindestens einem Monomeren abgeleitet sind, welches aus der Reihe der ungesättigten Carbonsäuren und deren Derivaten sowie ungesättigten Sulfonsäuren ausgewählt wurde.

**5.** Verwendung gemäß dem vorhergehenden Anspruch, dadurch gekennzeichnet, daß die ungesättigten Carbonsäuren und deren Derivate
- ungesättigte Monocarbonsäuren, sowie
- ungesättigte Dicarbonsäuren, deren Monoester sowie deren Monoamide aufweisen.

**6.** Verwendung gemäß dem vorhergehenden Anspruch, dadurch gekennzeichnet, daß die ungesättigten Carbonsäuren aus der Reihe Acrylsäure, Methacrylsäure sowie Crotonsäure und/oder die Dicarbonsäuren aus der Reihe Malein-, und Itaconsäure und/oder die Monoester oder Monoamide von den entsprechenden Alkoholen oder Aminen mit jeweils 1 bis 22 Kohlenstoffatome ausgewählt werden.

**7.** Verwendung gemäß einem der Ansprüche 2 bis 4, dadurch gekennzeichnet, daß die ungesättigten Sulfonsäuren aus der Reihe Methyl-2-acrylamido-2-propansulfonsäure und dem 2-Sulfoethylmethacrylat ausgewählt wurden.

**8.** Verwendung gemäß einem der vorhergehenden Ansprüche, dadurch gekennzeichnet, daß die Einheiten B, welche von mindestens einem hydrophilen Monomeren abgeleitet wurden, eine Amingruppe aufweisen, welche der folgenden Formel entspricht:

$$-\!\!\left[CH_2C(R_1)\right]\!\!-$$
$$CO{-}M{-}(CH_2)_n{-}N(R_2)(R_3)$$

worin:

| | |
|---|---|
| M | -O- oder -NH-, |
| $R_1$ | -H oder $-CH_3$, |
| n | eine Zahl zwischen 2 und 20 bedeuten, und |
| $R_2$ und $R_3$ | unabhängig voneinander -H oder eine Kohlenwasserstoffgruppe mit 1 bis 4 Kohlenstoffatomen darstellen. |

**9.** Verwendung gemäß vorhergehendem Anspruch, dadurch gekennzeichnet, daß die Einheiten B aus der Reihe der Monomere Dimethylaminoethylmethacrylat, Diethylaminoethylacrylat und N-Dimethylaminopropylmethacrylamid ausgewählt werden.

**10.** Verwendung gemäß einem der vorhergehenden Ansprüche, dadurch gekennzeichnet, daß die Einheiten B von mindestens einem hydrophilen ungesättigten Monomer mit einer Amidgruppierung abgeleitet sind, welch letztere aus der Reihe Acrylamid, Methacrylamid, N,N-Dimethylacrylamid, N-Vinylpyrrolidon, und Diacetonacrylamid ausgewählt wurde.

**11.** Verwendung gemäß einem der vorhergehenden Ansprüche, dadurch gekennzeichnet, daß die Copolymeren ein Molekulargewicht von mindestens oder gleich 200 000 aufweisen.

**12.** Verwendung gemäß einem der vorhergehenden Ansprüche, dadurch gekennzeichnet, daß das jeweilige Gewichtsverhältnis in bezug auf das erste und zweite Copolymere bei dieser Kombination im Bereich zwischen 10 : 90 und 90 : 10 und insbesondere im Bereich zwischen 25 : 75 und 75 : 25 liegt.

**13.** Verwendung gemäß dem vorhergehenden Anspruch, dadurch gekennzeichnet, daß die Gesamtkonzentration an dem ersten und zweiten Copolymeren in dieser Zubereitung weniger als 10 Gew.-% und insbesondere weniger als 5 Gew.-% beträgt.

**14.** Verwendung gemäß dem vorhergehenden Anspruch, dadurch gekennzeichnet, daß die Gesamtkonzentration an dem Copolymeren mehr als 0,1 Gew.-% beträgt.

**15.** Verwendung gemäß einem der vorhergehenden Ansprüche, dadurch gekennzeichnet, daß die Ölphase Kohlenwasserstoffe, Triglyzeride oder Säureester und/oder Ester aus langkettigen Alkoholen aufweist.

**16.** Verwendung gemäß einem der vorhergehenden Ansprüche, dadurch gekennzeichnet, daß jedes der Copolymeren zur Verdickung in einem Teil des Öls oder in einem Ölgemisch aufgelöst wird, und im Anschluß daran die beiden so erhaltenen Lösungen miteinander vermischt werden.

**17.** Verwendung gemäß Anspruch 16, dadurch gekennzeichnet, daß die öllöslichen Hilfsstoffe der Zubereitung vor oder nach der Zugabe des Copolymeren zu dem einen und/oder anderen Teil des Öls vor der Durchführung der Vermischung hinzugefügt werden.

**18.** Verwendung gemäß einem der vorhergehenden Ansprüche, dadurch gekennzeichnet, daß der Ölphase zusätzlich noch ein rheologisches Modifizierungsmittel einverleibt wird.

**19.** Verwendung gemäß dem vorhergehenden Anspruch, dadurch gekennzeichnet, daß das rheologische Modifizierungsmittel durch eine nichtionische, amphiphile Substanz gebildet wird.

**20.** Verwendung gemäß dem vorhergehenden Anspruch, dadurch gekennzeichnet, daß die amphiphile Substanz einen HLB-Wert zwischen 12 und 40 aufweist.

**21.** Verwendung gemäß einem der Ansprüche 18 bis 20, dadurch gekennzeichnet, daß das rheologische Modifizierungsmittel aus der Reihe

- Ester aus Fettsäuren und Polyoxyethylensorbitan,
- Ester aus Fettsäuren und Polyoxyethylenglycerin,
- Ester aus Fettsäuren und polyoxyethyliertem Propylenglykol,
- polyoxyethylierte oder polyoxypropylierte Alkylether,
- polyoxyethylierte oder polyoxypropylierte Alkylphenylether, oder
- polyoxyethylierte Guerbet-Alkohole

ausgewählt wurde.

**22.** Verwendung gemäß einem der Ansprüche 19 bis 21, dadurch gekennzeichnet, daß die amphiphile Substanz in hydratisierter Form eingesetzt wird.

**23.** Verwendung gemäß dem vorhergehenden Anspruch, dadurch gekennzeichnet, daß die amphiphile Substanz zusätzlich zur Anwesenheit eines wasserlöslichen Alkohols zum Einsatz gelangt.

**24.** Kosmetische Zubereitung, dadurch gekennzeichnet, daß sie eine verdickte Ölphase gemäß Definition nach einem der Ansprüche 1 bis 23 aufweist.

**25.** Kosmetische Zubereitung gemäß dem vorhergehenden Anspruch, dadurch gekennzeichnet, daß sie zusätzlich noch ein rheologisches Modifizierungsmittel enthält.

**26.** Kosmetische Zubereitung gemäß dem vorhergehenden Anspruch, dadurch gekennzeichnet, daß das rheologische Modifizierungsmittel der in einem der Ansprüche 19 bis 23 gegebenen Definition entspricht.

**27.** Kosmetische Zubereitung gemäß dem vorhergehenden Anspruch, dadurch gekennzeichnet, daß die Ölphase in Gewichtsprozent in bezug auf das Gesamtgewicht von 100 % die folgenden Komponenten enthält:

- Verdickungsmischung aus Copolymeren: 0,5 bis 10 % (vorzugsweise 2 bis 4 %);
- nichtionische amphiphile Substanz: 1 bis 10 % (vorzugsweise 4 bis 6 %);

- Wasser: 1 bis 10 % (vorzugsweise 4 bis 6 %);
- Öl(e): 55 bis 95 % (vorzugsweise 75 bis 85 %); sowie gegebenenfalls
- wasserlöslicher Alkohol: 1 bis 10 % (vorzugsweise 2 bis 6 %).

**28.** Kosmetische Zubereitung gemäß einem der Ansprüche 24 bis 27, dadurch gekennzeichnet, daß sie in Form einer Emulsion vorliegt.